# EUROPEAN PATENT SPECIFICATION

(11) **EP 1 082 128 B1**
(45) Date of publication and mention of the grant of the patent: **28.12.2005**
(21) Application number: 99926472.4
(22) Date of filing: 28.05.1999
(51) Int. Cl.: A61K 38/08, C07K 7/06, A61P 9/00

(54) **LYSINE CONTAINING PEPTIDES FOR TREATMENT OF HEART DISEASE**
LYSIN ENTHALTENDE PEPTIDE ZUR BEHANDLUNG VON HERZKRANKHEITEN
PEPTIDES CONTENANT DE LA LYSINE POUR LE TRAITEMENT DES TROUBLES CARDIAQUES

(30) Priority: 03.06.1998 US 89955
(43) Date of publication of application: 14.03.2001
(60) Divisional application: 04077333.5
(73) Proprietor: Ardana Bioscience Limited, Edinburgh EH3 7HA (GB)
(72) Inventor: Romano DEGHENGHI, 1264 St. Cergue (CH)
(74) Representative: Miles, John Stephen
(86) International application number: PCT/EP1999/003731
(87) International publication number: WO 1999/062539

(56) References cited:
- EP-A- 0 898 963
- WO-A-97/22620
- WO-A-98/22124
- R. DEGHENGHI: "Structural requirements of growth hormone secretagogues" GROWTH HORM. SECRETAGOGUES CLIN. PRACT. INT SYMP GH SECRETAGOGUES. 2ND,1997, pages 27-35, XP002118401
- R. DEGHENGHI ET AL.: "GH-releasing activity of hexarelin, a new growth hormon releasing peptide, in infant and adult rats" LIFE SCIENCES, vol. 54, no. 18, 1994, pages 1321-1328, XP002118642
- DEGHENGHI ET AL: "The development of 'impervious peptides' as growth hormone secretagogues" ACTA PAEDIATRICA. SUPPLEMENT, vol. 423, November 1997 (1997-11), pages 85-87, XP002118402 cited in the application
- LIHU Y ET AL: "Potent 3-spiropiperidine growth hormone secretagogues" BIOORGANIC & MEDICINAL CHEMISTRY LETTERS, vol. 8, no. 1-6, 6 January 1998 (1998-01-06), page 107-112 XP004136632 ISSN: 0960-894X
- COLONNA ET AL: 'CARDIAC ISCHEMIA AND IMPAIRMENT OF VASCULAR ENDOTHELIUM FUNCTIO INHEARTS FROM GROWTH HORMONE-DEFICIENT RATS: PROTECTION BY HEXARELIN' EUROPEAN JOURNAL OF PHARMACOLOGY no. 334, 1997, pages 201 - 207, XP000881672

## Description

The present invention relates to the use of certain lysine containing peptides to normalize cardiac pressure and to treat heart disease.

Under the general term heart disease, a variety of cardiac ailments, including myocardial ischemia, heart failure and related vascular dysfunction, are treated with drugs such as organic nitrates, calcium channel blockers, β-adrenergic receptor antagonists, antiplatelet and antithrombotic agents, cardiac glycosides, angiotensin converting enzyme inhibitors and angiotensin receptor antagonists. A general review of the field is found, for example, in Goodman & Gilman's "The Pharmacologic Basis of Therapeutics", IX edition, McGraw-Hill, New York, (1996), chapters 32 and 34.

Recently, the protective effect of a peptide known as Hexarelin (also called examorelin) having the structure His-D-2-methyl-Trp-Ala-Trp-D-Phe-Lys-NH₂ was described in an article by V. De Gennaro Colonna et al., European J. Pharmacology, 334, (1997), 201-207. Hexarelin was found to reverse the worsening of cardiac dysfunction in growth hormone deficient rats. At least part of its beneficial effect on myocardial ischemia was attributed to the growth hormone liberating properties of the peptide.

Heart disease is an increasing health problem as the population at large ages, such that there is a need for additional drugs or agents for treatment of these conditions. The present invention provides a number of peptides that are useful for this purpose.

WO98/22124 relates to the use of growth hormone (GH) secretagogue compound or GH and adrenocorticotropic hormone (ACTH) secretagogue compound for the manufacture of a medicament for treating cardiac failure or related vascular dysfunction. R. Deghenghi, Growth Hormone Secretagogues Clin. Pract. Int Symp GH Secretagogues, 2^{nd}, 1997, pages 27-35, reviews the discovery and development of peptide and nonpeptide synthetic secretagogues and their biologic properties. EP898963 discloses synthetic growth hormone secretagogues and their use in the treatment of congestive heart failure. Deghenghi et al., Life Sciences, vol. 54, no. 18, 1994, pages 1321-1328, disclose the GHRP analog hexarelin, a hexapeptide active in stimulating GH secretion. WO97/22620 discloses oligopeptides containing D2-alkyltryptophan capable of promoting the release of growth hormone which are active by the oral route. Deghenghi et al., Acta Pediatrica, Supplement 423: 85-87 (1997), reviews the discovery and development of growth hormone (GH) secretagogues. Lihu Y et al., Bioorganic & Medicinal Chemistry Letters, vol. 8, no. 1-6, pp. 107-112 (1998), describes SAR studies of regioisomers and homologues of the growth hormone secretagogue L-162,752. Colonna et al., European Journal of Pharmacology 334 (1997), 201-207, disclose the beneficial effects of hexarelin in cardiac dysfunction, such as myocardial ischemia and reperfusion damage.

The present invention relates to the use of a number of different lysine containing peptides to normalize cardiac pressure for treatment of heart disease conditions such as myocardial ischemia. The peptide sequences include a spirolactam, bicyclic or tricyclic peptidomimetic unit. The peptides disclosed herein exhibit binding to cardiac tissue and have been found to normalize cardiac pressure and to impart cardiac protecting activity. One common feature for all peptides is that at least one lysine unit is present.

According to the invention, there is provided the use of a peptide selected from the group consisting of:
His-D-Trp-D-Lys-Trp-D-Phe-Lys-NH₂,
His-D-Mrp-D-Lys-Trp-D-Phe-Lys-NH₂,
His-Ala-D-Trp-Lys-Mrp-D-Phe-Lys-NH₂,
His-D-Mrp-D-Lys-Mrp-D-Phe-Lys-NH₂,
His-Ala-D-Trp-Ala-Mrp-D-Phe-Lys-NH₂,
His-D-Trp-Ala-Mrp-D-Phe-Ala-Lys-NH₂,
[S,S-Spiro(Pro-Leu)]-D-Mrp-D-Trp-Phe-Lys-NH₂,
[S,S-Spiro(Pro-Leu)]-D-Mrp-Mrp-Lys-NH₂,
[S,S-Spiro(Pro-Leu)]-D-Mrp-Ala-Trp-D-Phe-Lys-NH₂,
[S,S-Spiro(Pro-Leu)]-D-Mrp-D-Lys-Trp-D-Phe-Lys-NH₂,
Tyr-[S,S-Spiro(Pro-Leu)]-D-Mrp-D-Lys-Trp-D-Phe-Lys-NH₂,
[S,S-Spiro(Pro-Ile)]-D-Mrp-D-Lys-Trp-D-Phe-Lys-NH₂,
[S, S-Spiro (Pro-Leu)]-D-Mrp-D-HNH-(SO₂CH₃)-Lys-NH₂,
HAIC-D-Mrp-D-Lys-Trp-D-Phe-Lys-NH₂,
ATAB-D-Mrp-D-Lys-Trp-D-Phe-Lys-NH₂
where: D is the Dextro enantiomer, Mrp is 2-Alkyl-Trp, where the Alkyl group has one to three carbon atoms, HAIC is (2S,5S)-5-amino-1,2,4,5,6,7-hexahydro-azepino[3,2,1-hi]indole-4-one-2-carboxylic acid, ATAB is 2-R(2β,5β,8β)-8-amino-7-oxo-4-thia-1-aza-bicyclo[3.4.0]nonan-2-carboxylic acid, S,S-Spiro(Pro-Leu) and S,S-Spiro(Pro-Ile) is 4-Methyl-2S[6¹-oxo(5¹-S)1¹, 7¹-diazaspiro[4,4]nonan-7¹-yl-]pentanoic acid of formula 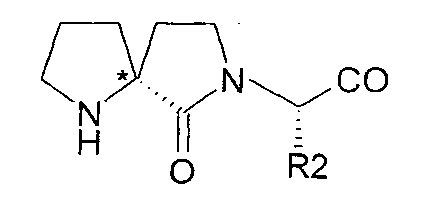 where R2 is the side chain of Leu or Ile (see P. Ward et al., *J. Med Chem*. 33, 1848 (1990),
   and Ala, Lys, Phe, Trp, His, Thr, Cys, Tyr, Leu and Ile are the amino acids Alanine, Lysine, Phenylalanine, Tryptophan, Histidine, Threonine, Cysteine, Tyrosine, Leucine and Isoleucine, respectively, for the preparation of a medicament for normalising cardiac pressure and/or for treating heart disease.

Pharmaceutically acceptable salts of the peptides of the present invention can be used, if desired. Such salts would include organic or inorganic addition salts, including hydrochloride, hydrobromide, phosphate, sulfate, acetate, succinate, ascorbate, tartrate, gluconate, benzoate, malate, fumarate, stearate and pamoate salts.

All these peptides can be conveniently synthesized according to the usual methods of peptide chemistry, such as by solid phase peptide synthesis, as described by E. Atherton and R. C. Sheppard in "Solid Phase Peptide Synthesis" IRL Press at Oxford University Press 1989, by solution phase synthesis as described by J. Jones in "The Chemical Synthesis of Peptides", Clarendon Press, Oxford 1994, or by both solid-and solution-phase methods, as known in the art.

The solid-phase synthesis starts from the C-terminal end of peptide. A suitable starting material can be prepared, for example, by attaching the required protected alpha-amino acid to a chloromethylated resin, a hydroxymethylated resin, a benzhydrylamine resin (BHA), or to a para-methylbenzhydrylamine resin (p-Me-BHA). As an example, an available chloromethylated resin is BIOBEADS® SX 1 by BioRad Laboratories, Richmond, California. The preparation of the hydroxymethyl resin is described by Bodansky et al., Chem. Ind. (London) 38, 15997 (1966). The BHA resin is described by Pietta and Marshall, Chem. Comm., 650 (1970) and is commercially available by Peninsula Laboratories Inc., Belmont, California.

After the starting attachment, the protecting group of the alpha-amino acid can be removed by means of different acid reagents, comprising trifluoroacetic acid (TFA) or hydrochloric acid (HC1) dissolved in organic solvents at room temperature. After the removal of the protecting group of the alpha-amino acid, the remaining protected amino acids can be coupled step by step in the desired order. Each protected amino acid can generally be reacted in excess of about three times using a suitable carboxyl activating group, such as dicyclohexylcarbodiimiide (DCC) or diisopropylcarbo-diimide (DIC) dissolved, for example, in methylene chloride (CH₂Cl₂), dimethylformamide (DMF) or their mixtures. After the desired aminoacidic sequence has been completed, the desired peptide can be cleaved from the supporting resin by treatment with a reagent such as hydrogen fluoride (HF) which cleaves not only the peptide from the resin, but also the protecting groups of the lateral chains. When a chloromethylated resin or a hydroxymethylated resin is used, the treatment with HF leads to the formation of the terminal acid peptide in free form. When a BHA or p-Me-BHA resin is used, treatment with HF directly leads to the formation of the terminal amide peptide in free form.

These medicaments useful for treating cardiac diseases in an animal, including a human, can comprise a peptide of the present invention or a pharmaceutically acceptable salt thereof, or combinations of peptides of the present invention or pharmaceutically acceptable salts thereof, optionally, in admixture with a carrier, excipient, vehicle, diluent, matrix or delayed release coating. Examples of such carriers, excipients, vehicles and diluents, can be found in Remington's Pharmaceutical Sciences, 18th Edition, A.R. Gennaro, Ed., Mack Publishing Company, Easton, PA, 1990. These medicaments can be administered to animals, including humans, at a therapeutically effective dose which can be easily determined by one of skill in the art and which can vary according to the specie, age, sex and weight of the treated patient or subject. For example, in humans, when intravenously administered, the preferred dose falls in the range from about 1 µg to about 25 µg of total peptide per kg of body weight. When orally administered, typically higher amounts are necessary. For example, in humans for the oral administration, the dosage level is typically from about 30 µg to about 1000 µg of polypeptide per kg of body weight. The exact level can be easily determined empirically based on the above disclosure.

Any of the peptides of the present invention can be formulated by the skilled in the art to provide medicaments which are suitable for parenteral, buccal, rectal, vaginal, transdermal, pulmonary or oral routes by adjusting the dose as needed, such doses being in the range of from about 1 µg/kg to 1 mg/kg of body weight as noted above.

These peptides are typically administered to mammals experiencing heart diseases where cardiac pressure has been reduced. Reduced cardiac pressure is encountered after infarctions, for example, as well as in other heart problems or conditions. These peptides work directly on the heart to cause cardiac pressure to be returned to substantially normal levels. The type of formulation of medicaments containing these peptides can be selected so that these peptides are rapidly delivered, e.g., by a nasal or intravenous route, when necessary.

### BRIEF DESCRIPTION OF THE DRAWINGS

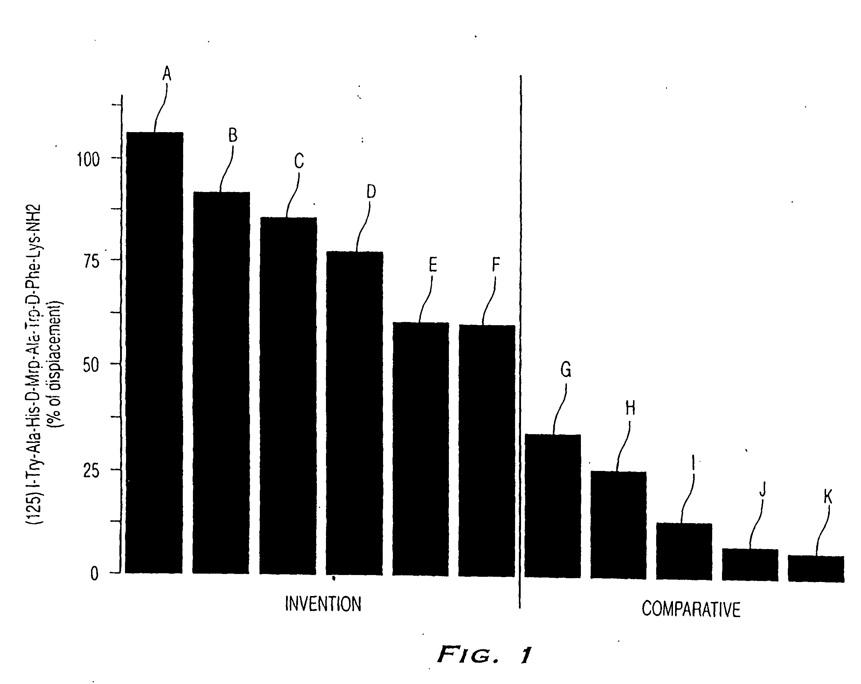

Figure 1 is a graphical illustration of the ability of certain peptides to bind to heart tissue.

### EXAMPLES

In the examples that follow, data is presented for the most preferred lysine containing peptides of the invention. The cardiac protection of the instant peptides has been measured essentially as described in the publication by V. De Gennaro Colonna et al., *Europ. J. Pharmacol.* 334:201-207 (1997).

### Example 1

The effects on coronary perfusion pressure ("CPP") in isolated rat hearts have been measured using Hexarelin (His-D-Mrp-Ala-Trp-D-Phe-Lys-NH₂) as reference compound and compared to that for the GH antagonist His-D-Mrp-D-Lys-Trp-D-Phe-Lys-NH₂. An increase of CPP over a saline treatment (i.e., no peptide) of 110 ± 10% was found for Hexarelin, but an increase of 160 ± 25% was found for the antagonist. This is a totally unexpected and surprising finding, since the antagonist does not liberate GH.

### Example 2

The binding abilities of lysine containing peptides compared to non-lysine containing peptides on human heart membranes are shown in Figure 1. These data have been obtained according to the method of G. Muccioli et. al., *J. Endocrinology,* 156, 90 (1998). Data for the peptides used are show in the graph using the following identifications. No. peptide
- A: His-D-Mrp-D-Lys-Trp-D-Phe-Lys-NH₂
- B: [Spiro (S,S)-(Pro-Leu)]-D-Mrp-D-Trp-Phe-Lys-NH₂
- C: D-Mrp-D-Mrp-Phe-NH₂
- D: GAB-D-Mrp-D-Mrp-NH₂
- E: D-Mrp-Mrp-NH₂
- F: AIB-D-Mrp-Mrp-NH₂
- G: AIB-D-Mrp-D-Mrp-NH₂

Peptides A-B are in accordance with the invention, while peptides C-G are comparative. As shown in the figure, lysine containing peptides provided inhibition (i.e., displacement) of ¹²⁵I-Tyr-Ala-His-D-Mrp-Ala-Trp-D-Phe-Lys-NH₂ in proportions of over 60 to over 100%, whereas non-lysine containing peptides G-K only provided about 5 to less than 35%. The greater binding affinities for the lysine containing peptides of the invention illustrate that these peptides directly operate on specific receptors of heart tissue to achieve normalization of cardiac pressure.

### Examples 3-4

These examples illustrate preferred formulations for administration of the lysine containing peptides of the invention.

### Example 3

The peptide His-D-Mrp-D-Lys-Trp-D-Phe-Lys-NH₂ is lyophilized in sterile vials containing 100 micrograms of the peptide and 10 mg of mannitol as excipient. Water for injection is then used to dissolve the peptide into a formulation which can be injected i.v. into mammals with impaired cardiac function at a dose of 1 µg/kg body weight.

### Example 4

The peptide of Example 3 is dissolved in sterile water containing 0.05% of chlorocresol as a preservative. This solution can be administered intranasally at doses of 20 to 60 µg/kg twice or three times daily to mammals with impaired heart function so that the peptides can be rapidly absorbed.

## Claims

1. The use of a peptide selected from the group consisting of:
His-D-Trp-D-Lys-Trp-D-Phe-Lys-NH₂,
His-D-Mrp-D-Lys-Trp-D-Phe-Lys-NH₂,
His-Ala-D-Trp-Lys-Mrp-D-Phe-Lys-NH₂,
His-D-Mrp-D-Lys-Mrp-D-Phe-Lys-NH₂,
His-Ala-D-Trp-Ala-Mrp-D-Phe-Lys-NH₂,
His-D-Trp-Ala-Mrp-D-Phe-Ala-Lys-NH₂,
[S,S-Spiro(Pro-Leu)]-D-Mrp-D-Trp-Phe-Lys-NH₂,
[S,S-Spiro(Pro-Leu)]-D-Mrp-Mrp-Lys-NH₂,
[S,S-Spiro(Pro-Leu)]-D-Mrp-Ala-Trp-D-Phe-Lys-NH₂,
[S,S-Spiro(Pro-Leu)]-D-Mrp-D-Lys-Trp-D-Phe-Lys-NH₂,
Tyr-[S,S-Spiro(Pro-Leu)]-D-Mrp-D-Lys-Trp-D-Phe-Lys-NH₂,
[S,S-Spiro(Pro-Ile)]-D-Mrp-D-Lys-Trp-D-Phe-Lys-NH₂,
[S,S-Spiro(Pro-Leu)]-D-Mrp-D-HNH-(SO₂CH₃)-Lys-NH₂,
HAIC-D-Mrp-D-Lys-Trp-D-Phe-Lys-NH₂,
ATAB-D-Mrp-D-Lys-Trp-D-Phe-Lys-NH₂,
where: D is the dextro enantiomer, Mrp is 2-Alkyl-Trp, where the alkyl group has one to three carbon atoms, HAIC is (2S,5S)-5-amino-1,2,4,5,6,7-hexahydro-azepino[3,2,1-hi]indole-4-one-2-carboxylic acid, ATAB is 2-R(2β,5β,8β)-8-amino-7-oxo-4-thia-1-aza-bicyclo[3.4.0]nonan-2-carboxylic acid, S,S-Spiro(Pro-Leu) and S,S-Spiro(Pro-Ile) is 4-Methyl-2S[6¹-oxo(5¹-S)1¹,7¹-diazaspiro[4,4]nonan-7¹-yl-]pentanoic acid of formula 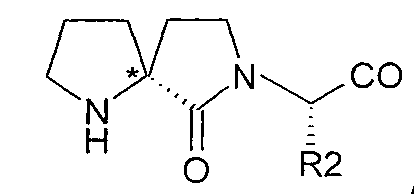 where R2 is the side chain of Leu or Ile; for the preparation of a medicament for normalising cardiac pressure and/or for treating heart disease.

2. The use of a peptide according to claim 1 wherein the heart disease is myocardial ischemia, impaired cardiac function or heart failure.

3. The use according to claim 1 or claim 2, in which the medicament is in injectable form.

4. The use according to claim 1 or claim 2, wherein the medicament is for buccal, rectal, vaginal, transdermal, pulmonary, nasal or oral administration.

## Patentansprüche

1. Verwendung eines Peptids, das aus der Gruppe ausgewählt ist, welche besteht aus:
His-D-Trp-D-Lys-Trp-D-Phe-Lys-NH₂,
His-D-Mrp-D-Lys-Trp-D-Phe-Lys-NH₂,
His-Ala-D-Trp-Lys-Mrp-D-Phe-Lys-NH₂,
His-D-Mrp-D-Lys-Mrp-D-Phe-Lys-NH₂,
His-Ala-D-Trp-Ala-Mrp-D-Phe-Lys-NH₂,
His-O-Trp-Ala-Mrp-D-Phe-Ala-Lys-NH₂,
[S, S-Spiro (Pro-Leu)]-D-Mrp-D-Trp-Phe-Lys-NH₂.
[S, S-Spiro (Pro-Leu)]-D-Mrp-Mrp-Lys-NH₂,
[S, S-Spiro (Pro-Leu)]-D-Mrp-Ala-Trp-D-Phe-Lys-NH₂,
[S, S-Spiro (Pro-Leu)]-D-Mrp-D-Lys-Trp-D-Phe-Lys-NH₂.
Tyr- [S, S-Spiro (Pro-Leu)]-D-Mrp-D-Lys-Trp-D-Phe-Lys-NH₂,
[S, S-Spiro (Pro-Ile)]-D-Mrp-D-Lys-Trp-D-Phe-Lys-NH₂,
[S, S-Spiro (Pro-Leu)]-D-Mrp-D-HNH-(SO₂CH₃)-Lys-NH₂,
HAIC-D-Mrp-D-Lys-Trp-D-Phe-Lys-NH₂,
ATAB-D-Mrp-D-Lys-Trp-D-Phe-Lys-NH₂.
wobei: D das rechtsdrehende Enantiomer ist, Mrp 2-Alkyl-Trp ist, wobei die Akylgruppe 1 bis 3 Kohlenstoffatome aufweist, HAIC (2S, 5S)-5-Amino-1,2,4,5,6,7-Hexahydroazepino-[3,2,1-hi]-Indol-4-on-2-Karbonsäure ist, ATAB 2-R-(2β, 5β, 8β)-8-Amino-7-Oxo-4-Thia-1-Aza-Bizyklo-[3.4.0]-Nonan-2-Karbonsäure ist, S, S-Spiro-(Pro-Leu) und S, S-Spiro(Pro-Ile) 4-Methyl-2S-[6¹-Oxo-(5¹-S)-1¹,7¹-Diazaspiro-[4,4]-Nonan-7¹-yl-]-Pentansäure der Formel 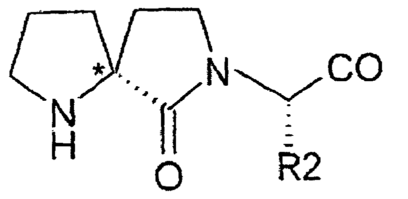 ist, wobei R2 die Leu- oder lie-Seitenkette ist,
für die Herstellung eines Medikaments zum Normalisieren des Blutdrucks und für die Behandlung einer Herzerkrankung.

2. Verwendung eines Peptids nach Anspruch 1, wobei die Herzerkrankung myokardiale lschämie, unzureichende Herzfunktion oder Herzinfarkt ist.

3. Verwendung nach Anspruch 1 oder 2, wobei das Medikament injizierbare Form hat.

4. Verwendung nach Anspruch 1 oder 2, wobei das Medikament für die bukkale, rektale, vaginale, transdermale, pulmonare, nasale oder orale Verabreichung vorgesehen ist.

## Revendications

1. Utilisation d'un peptide choisi dans le groupe constitué de :
His-D-Trp-D-Lys-Trp-D-Phs-Lys-NH₂,
His-D-Mrp-D-Lys-Trp-D-Phe-Lys-NH₂,
His-Ala-D-Trp-Tys-Mrp-D-Phe-Lys-NH₂,
His-D-Mrp-D-Lys-Mrp-D-Phe-Lys-NH₂,
His-Ala-D-Trp-Ala-Mrp-D-Phe-Lys-NH₂,
His-D-Trp-Ala-Mrp-D-Phe-Ala-Lys-NH₂,
[S,S-Spiro(Pro-Leu)]-D-Mrp-D-Trp-Phe-Lys-NH₂,
[S,S-Spiro(Pro-Leu)]-D-Mrp-Mrp-Lys-NH₂,
[S,S-Spiro(Pro-Leu)]-D-Mrp-Ala-Trp-D-Phe-Lys-NH₂,
[S,S-Spiro(Pro-Leu)]-D-Mrp-D-Lys-Trp-D-Phe-Lys-NH₂,
Tyr-[S,S-Spiro(Pro-Leu)]-D-Mrp-D-Lys-Trp-D-Phe-Lys-NH₂,
[S,S-Spiro(Pro-Ile)]-D-Mrp-D-Lys-Trp-D-Phe-Lys-NH₂,
[S,S-Spiro(Pro-Leu)]-D-Mrp-D-HNH-(SO₂CH₃)-Lys-NH₂,
HAIC-D-Mrp-D-Lys-Trp-D-Phe-Lys-NH₂,
ATAB-D-Mrp-P-Lys-Trp-D-Phe-Lys-NH₂.
où : D est l'énantiomère dextro, Mrp est le 2-alkyl-Trp, où le groupe alkyle possède un à trois atomes de carbone, HAIC est l'acide (2S,5S)-5-amino-1,2,4,5,6,7-hexahydro-azépino[3,2,1-hi]indole-4-one-2-carboxylique, ATAB est l'acide carboxylique 2-R(2β, 5β, 8β)-8-amino-7-oxo-4-thia-1-aza-bicyclo[3.4.0]nonan-2, le S,S-spiro(Pro-leu) et le S,S-spiro(Pro-Ile) sont l'acide pentanoïque 4-méthyl-2S [6¹-oxo(5¹-S)1¹,7¹-diazospiro[4,4]nonan-7¹-yl-] de formule : 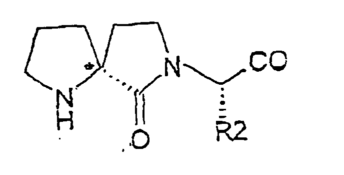 où R2 est la chaîne latérale de Leu ou Ile ; pour la préparation d'un médicament pour la normalisation de la pression cardiaque ou pour le traitement de maladies cardiaques.

2. Utilisation d'un peptide selon la revendication 1, dans laquelle la maladie cardiaque est une ischémie, une fonction cardiaque altérée ou une insuffisance cardiaque.

3. Utilisation d'un peptide selon la revendication 1 ou la revendication 2 dans laquelle le médicament est sous forme injectable.

4. Utilisation d'un peptide selon la revendication 1 ou la revendication 2, dans laquelle le médicament est pour une administration par voie buccale, rectale, vaginale, transdermique, pulmonaire, nasale ou orale.


### SEQUENCE LISTING

<110> Deghenghi, Romano
<120> Lysine containing peptides for treatment of heart disease
<130> deghenghi
<140>
   <141>
<160> 39
<170> PatentIn Ver. 2.1
<210> 1
   <211> 6
   <212> PRT
   <213> Artificial Sequence
<220>
   <223> Description of Artificial Sequence:synthetic peptides
<220>
   <221> MOD_RES
   <222> (2)
   <223> Trp is D-TRP
<220>
   <221> MOD_RES
   <222> (4)
   <223> Phe is D-Phe
<400> 1
   His Trp Ala Trp Phe Lys
    1               5
<210> 2
   <211> 6
   <212> PRT
   <213> Artificial Sequence
<220>
   <223> Description of Artificial Sequence:synthetic peptides
<220>
   <221> MOD_RES
   <222> (2)
   <223> Trp is D-Trp
<220>
   <221> MOD_RES
   <222> (5)
   <223> Phe Is D-Phe
<220>
   <221> MOD_RES
   <222> (4)
   <223> Trp Is 2-alkyl-Trp
<400> 2
   His Trp Ala Trp Phe Lys
   1 5
<210> 3
   <211> 7
   <212> PRT
   <213> Artificial Sequence
<220>
   <223> Description of Artificial Sequence:synthetic peptides
<220>
   <221> MOD_RES
   <222> (1)
   <223> Thr is D-Thr
<220>
   <221> MOD_RES
   <222> (3)
   <223> Trp is D- 2-alkyl-Trp
<220>
   <221> MOD_RES
   <222> (6)
   <223> Phe is D-Phe
<400> 3
   Thr His Trp Ala Trp Phe Lys
   1 5
<210> 4
   <211> 6
   <212> PRT
   <213> Artificial Sequence
<220>
   <223> Description of Artificial Sequence:synthetic peptides
<220>
   <221> MOD_RES
   <222> (2)
   <223> Trp is D- 2-alkyl- Trp
<220>
   <221> MOD_RES
   <222> (5)
   <223> Phe is D-Phe
<400> 4
   Thr Trp Ala Trp Phe Lys
   1 5
<210> 5
   <211> 4
   <212> PRT
   <213> Artificial Sequence
<220>
   <223> Description of Artificial Sequence:synthetic peptides
<220>
   <221> MOD_RES
   <222> (1)
   <223> Trp is IMA-D-2-alkyl-Trp
<220>
   <221> MOD_RES
   <222> (2)
   <223> Trp is D-Trp
<400> 5
   Trp Trp Phe Lys
   1
<210> 6
   <211> 4
   <212> PRT
   <213> Artificial Sequence
<220>
   <223> Description of Artificial Sequence:synthetic peptides
<220>
   <221> MOD_RES
   <222> (1)
   <223> Trp is IMA-D-2-alkyl-Trp
<220>
   <221> MOD_RES
   <222> (2)
   <223> Ala is D-Naphthyl-Ala
<400> 6
   Trp Ala Phe Lys
   1
<210> 7
   <211> 4
   <212> PRT
   <213> Artificial Sequence
<220>
   <223> Description of Artificial Sequence:synthetic peptides
<220>
   <221> MOD_RES
   <222> (1)
   <223> Trp is GAB-D-2-alkyl-Trp
<220>
   <221> MOD_RES
   <222> (2)..(3)
   <223> Trp is D-Trp
<400> 7
   Trp Trp Trp Lys
   1
<210> 8
   <211> 6
   <212> PRT
   <213> Artificial Sequence
<220>
   <223> Description of Artificial Sequence:synthetic peptides
<220>
   <221> MOD_RES
   <222> (1)
   <223> Ala is D-Ala
<220>
   <221> MOD_RES
   <222> (2)
   <223> Ala is D-naphthyl-Ala
<220>
   <221> MOD_RES
   <222> (5)
   <223> Phe is D-Phe
<400> 8
   Ala Ala Ala Trp Phe Lys
   1 5
<210> 9
   <211> 4
   <212> PRT
   <213> Artificial Sequence
<220>
   <223> Description of Artificial Sequence:synthetic peptides
<220>
   <221> MOD_RES
   <222> (1)
   <223> Ala is INIP-D-Naphthyl-Ala
<220>
   <221> MOD_RES
   <222> (2)
   <223> Ala is D-naphthyl-Ala
<400> 9
   Ala Ala Phe Lys
   1
<210> 10
   <211> 4
   <212> PRT
   <213> Artificial Sequence
<220>
   <223> Description of Artificial Sequence:synthetic peptides
<220>
   <221> MOD_RES
   <222> (1)
   <223> Ala is INIP- D-naphthyl-Ala
<220>
   <221> MOD_RES
   <222> (2)
   <223> Trp is D-Trp
<400> 10
   Ala Trp Phe Lys
   1
<210> 11
   <211> 5
   <212> PRT
   <213> Artificial Sequence
<220>
   <223> Description of Artificial Sequence:synthetic peptides
<220>
   <221> MOD_RES
   <222> (1)
   <223> Trp is IMA-D-2-alkyl-Trp
<220>
   <221> MOD_RES
   <222> (4)
   <223> Phe is D-Phe
<400> 11
   Trp Ala Trp Phe Lys
   1 5
<210> 12
   <211> 4
   <212> PRT
   <213> Artificial Sequence
<220>
   <223> Description of Artificial Sequence:synthetic peptides
<220>
   <221> MOD_RES
   <222> (1)
   <223> Trp is INIP-D-2-alkyl-Trp
<220>
   <221> MOD_RES
   <222> (2)
   <223> Trp is D-Trp
<400> 12
   Trp Trp Phe Lys
   1
<210> 13
   <211> 4
   <212> PRT
   <213> Artificial Sequence
<220>
   <223> Description of Artificial Sequence:synthetic peptides
<220>
   <221> MOD_RES
   <222> (1)
   <223> Trp is INIP-D-2-alkyl-Trp
<220>
   <221> MOD_RES
   <222> (2)
   <223> Ala is D-naphthyl-Ala
<400> 13
   Trp Ala Phe Lys
   1
<210> 14
   <211> 4
   <212> PRT
   <213> Artificial Sequence
<220>
   <223> Description of Artificial Sequence:synthetic peptides
<220>
   <221> MOD_RES
   <222> (1)
   <223> Trp is GAB-D-2-alkyl-Trp
<220>
   <221> MOD_RES
   <222> (2)
   <223> Trp is D-Trp
<400> 14
   Trp Trp Phe Lys
   1
<210> 15
   <211> 4
   <212> PRT
   <213> Artificial Sequence
<220>
   <223> Description of Artificial Sequence:synthetic peptides
<220>
   <221> MOD_RES
   <222> (1)
   <223> Trp is TXM-D-2-alkyl-Trp
<220>
   <221> MOD_RES
   <222> (2)
   <223> Trp is D-Trp
<400> 15
   Trp Trp Phe Lys
   1
<210> 16
   <211> 4
   <212> PRT
   <213> Artificial Sequence
<220>
   <223> Description of Artificial Sequence:synthetic peptides
<220>
   <221> MOD_RES
   <222> (1)
   <223> Trp is GAB-D-2-alkyl-Trp
<220>
   <221> MOD_RES
   <222> (2)
   <223> Trp is 2-alkyl-Trp
<400> 16
   Trp Trp Phe Lys
   1
<210> 17
   <211> 7
   <212> PRT
   <213> Artificial Sequence
<220>
   <223> Description of Artificial Sequence:synthetic peptides
<220>
   <221> MOD_RES
   <222> (3)
   <223> Trp is D-2-alkyl-Trp
<220>
   <221> MOD_RES
   <222> (6) <223> Phe is D-Phe
<400> 17
   Ala His Trp Ala Trp Phe Lys
   1 5
<210> 18
   <211> 7
   <212> PRT
   <213> Artificial Sequence
<220>
   <223> Description of Artificial Sequence:synthetic peptides
<220>
   <221> MOD_RES
   <222> (2)
   <223> Trp is D-2-alkyl-trp
<220>
   <221> MOD_RES
   <222> (5)
   <223> Phe is D-Phe
<400> 18
   His Trp Ala Trp Phe Lys Thr
   1 5
<210> 19
   <211> 7
   <212> PRT
   <213> Artificial Sequence
<220>
   <223> Description of Artificial Sequence:synthetic peptides
<220>
   <221> MOD_RES
   <222> (2)
   <223> trp is D-2-alkyl-Trp
<220>
   <221> MOD_RES
   <222> (5)
   <223> Phe is D-Phe
<220>
   <221> MOD_RES
   <222> (7)
   <223> Thr is D-Thr
<400> 19
   His Trp Ala Trp Phe Lys Thr
   1 5
<210> 20
   <211> 6
   <212> PRT
   <213> Artificial Sequence
<220>
   <223> Description of Artificial Sequence:synthetic peptides
<220>
   <221> MOD_RES
   <222> (1)
   <223> Thr is D-Thr
<220>
   <221> MOD_RES
   <222> (2)
   <223> Trp is D-2-alkyl-Trp
<220>
   <221> MOD_RES
   <222> (5)
   <223> Phe is D-Phe
<400> 20
   Thr Trp Ala Trp Phe Lys
   1 5
<210> 21
   <211> 4
   <212> PRT
   <213> Artificial Sequence
<220>
   <223> Description of Artificial Sequence:synthetic peptides
<220>
   <221> MOD_RES
   <222> (1)
   <223> Trp is GAB-D-2-alkyl-Trp
<220>
   <221> MOD_RES
   <222> (2)
   <223> Ala is D-naphthyl-Ala
<400> 21
   Trp Ala Phe Lys
   1
<210> 22
   <211> 4
   <212> PRT
   <213> Artificial Sequence
<220>
   <223> Description of Artificial Sequence:synthetic peptides
<220>
   <221> MOD_RES
   <222> (1)
   <223> Trp is GAB-D-2-alkyl-Trp
<220>
   <221> MOD_RES
   <222> (2)
   <223> Trp is D-2-alkyl-Trp
<220>
   <221> MOD_RES
   <222> (4)
   <223> Trp is 2-alkyl-Trp
<220>
   <221> MOD_RES
   <222> (3)
   <223> Trp Is 2-alkyl-Trp
<400> 22
   Trp Trp Trp Lys
   1
<210> 23
   <211> 6
   <212> PRT
   <213> Artificial Sequence
<220>
   <223> Description of Artificial Sequence:synthetic peptides
<220>
   <221> MOD_RES
   <222> (3)
   <223> Trp is GAB-D-2-alkyl-Trp
<220>
   <221> MOD_RES
   <222> (4)
   <223> Trp is D-2-alkyl-Trp
<220>
   <221> MOD_RES
   <222> (5)
   <223> Trp is 2-alkyl-Trp
<400> 23
   Cys Tyr Trp Trp Trp Lys
   1 5
<210> 24
   <211> 8
   <212> PRT
   <213> Artificial Sequence
<220>
   <223> Description of Artificial Sequence:synthetic peptides
<220>
   <221> MOD_RES
   <222> (4)
   <223> Trp isD- 2-alkyl-Trp
<220>
   <221> MOD_RES
   <222> (7)
   <223> Phe is D-Phe
<400> 24
   Tyr Ala His Trp Ala Trp Phe Lys
   1 5
<210> 25
   <211> 6
   <212> PRT
   <213> Artificial Sequence
<220>
   <223> Description of Artificial Sequence:synthetic peptides
<220>
   <221> MOD_RES
   <222> (1)
   <223> Ala is D-Ala
<220>
   <221> MOD_RES
   <222> (2)
   <223> Trp is D-2-alkyl-Trp
<220>
   <221> MOD_RES
   <222> (5)
   <223> Phe is D-Phe
<400> 25
   Ala Trp Ala Trp Phe Lys
   1 5
<210> 26
   <211> 6
   <212> PRT
   <213> Artificial Sequence
<220>
   <223> Description of Artificial Sequence:synthetic peptides
<220>
   <221> MOD_RES
   <222> (2)
   <223> Trp is D-2-alkyl-Trp
<220>
   <221> MOD_RES
   <222> (3)
   <223> Lys is D-Lys
<220>
   <221> MOD_RES
   <222> (5)
   <223> Phe is D-Phe
<400> 26
   His Trp Lys Trp Phe Lys
   1 5
<210> 27
   <211> 7
   <212> PRT
   <213> Artificial Sequence
<220>
   <223> Description of Artificial Sequence:synthetic peptides
<220>
   <221> MOD_RES
   <222> (3)
   <223> Trp is D-Trp
<220>
   <221> MOD_RES
   <222> (5)
   <223> Trp is 2-alkyl-Trp
<220>
   <221> MOD_RES
   <222> (6)
   <223> Phe is D-Phe
<400> 27
   His Ala Trp Lys Trp Phe Lys
   1 5
<210> 28
   <211> 6
   <212> PRT
   <213> Artificial Sequence
<220>
   <223> Description of Artificial Sequence:synthetic peptides
<220>
   <221> MOD_RES
   <222> (2)
   <223> Trp is D-2-alkyl-Trp
<220>
   <221> MOD_RES
   <222> (3)
   <223> Lys is D-Lys
<220>
   <221> MOD_RES
   <222> (4)
   <223> Trp is 2-alkyl-Trp
<220>
   <221> MOD_RES
   <222> (5)
   <223> Phe is D-Phe
<400> 28
   His Trp Lys Trp Phe Lys
   1 5
<210> 29
   <211> 7
   <212> PRT
   <213> Artificial Sequence
<220>
   <223> Description of Artificial Sequence:synthetic peptides
<220>
   <221> MOD_RES
   <222> (3)
   <223> Trp is D-Trp
<220>
   <221> MOD_RES
   <222> (5)
   <223> Trp is 2-alkyl-Trp
<220>
   <221> MOD_RES
   <222> (6)
   <223> Phe is D-Phe
<400> 29
   His Ala Trp Ala Trp Phe Lys
   1 5
<210> 30
   <211> 7
   <212> PRT
   <213> Artificial Sequence
<220>
   <223> Description of Artificial Sequence:synthetic peptides
<220>
   <221> MOD_RES
   <222> (2)
   <223> Trp is D-Trp
<220>
   <221> MOD_RES
   <222> (4)
   <223> Trp is 2-alkyl-Trp
<220>
   <221> MOD_RES
   <222> (5)
   <223> Phe is D-Phe
<400> 30
   His Trp Ala Trp Phe Ala Lys
   1 5
<210> 31
   <211> 6
   <212> PRT
   <213> Artificial Sequence
<220>
   <223> Description of Artificial Sequence:synthetic peptides
<220>
   <221> MOD_RES
   <222> (1)..(2)
   <223> Pro-Leu is S,S-spiro(Pro-leu)
<220>
   <221> MOD_RES
   <222> (3)
   <223> Trp is D-2-alkyl-Trp
<220>
   <221> MOD_RES
   <222> (4)
   <223> Trp is D-Trp
<400> 31
   Pro Leu Trp Trp Phe Lys
   1 5
<210> 32
   <211> 5
   <212> PRT
   <213> Artificial Sequence
<220>
   <223> Description of Artificial Sequence:synthetic peptides
<220>
   <221> MOD_RES
   <222> (1)..(2)
   <223> Pro-Leu is S,S-spiro (Pro-Leu)
<220>
   <221> MOD_RES
   <222> (3)
   <223> Trp is d-2-alkyl-Trp
<220>
   <221> MOD_RES
   <222> (4)
   <223> Trp is 2-alkyl-Trp
<400> 32
   Pro Leu Trp Trp Lys
   1 5
<210> 33
   <211> 7
   <212> PRT
   <213> Artificial Sequence
<220>
   <223> Description of Artificial Sequence:synthetic peptides
<220>
   <221> MOD_RES
   <222> (1)..(2)
   <223> Pro-Leu is S,S-spiro(Pro-Leu)
<220>
   <221> MOD_RES
   <222> (3)
   <223> Trp is D-2-alkyl-Trp
<220>
   <221> MOD_RES
   <222> (6)
   <223> Phe is D-Phe
<400> 33
   Pro Leu Trp Ala Trp Phe Lys
   1 5
<210> 34
   <211> 7
   <212> PRT
   <213> Artificial Sequence
<220>
   <223> Description of Artificial Sequence:synthetic peptides
<220>
   <221> MOD_RES
   <222> (1)..(2)
   <223> Pro-Leu is S,S-spiro-(Pro-Leu)
<220>
   <221> MOD_RES
   <222> (3)
   <223> Trp is D-2-alkyl-trp
<220>
   <221> MOD_RES
   <222> (4)
   <223> Lys is D-Lys
<220>
   <221> MOD_RES
   <222> (6)
   <223> Phe is D-Phe
<400> 34
   Pro Leu Trp Lys Trp Phe Lys
   1 5
<210> 35
   <211> 8
   <212> PRT
   <213> Artificial Sequence
<220>
   <223> Description of Artificial Sequence:synthetic peptides
<220>
   <221> MOD_RES
   <222> (2)..(3)
   <223> Pro-Leu is S,S-spiro-(Pro-Leu)
<220>
   <221> MOD_RES
   <222> (4)
   <223> Trp is D-2-alkyl-Trp
<220>
   <221> MOD_RES
   <222> (5)
   <223> Lys is D-Lys
<220>
   <221> MOD_RES
   <222> (7)
   <223> Phe is D-Phe
<400> 35
   Tyr Pro Leu Trp Lys Trp Phe Lys
   1 5
<210> 36
   <211> 7
   <212> PRT
   <213> Artificial Sequence
<220>
   <223> Description of Artificial Sequence:synthetic peptides
<220>
   <221> MOD_RES
   <222> (1)..(2)
   <223> Pro-Ile is S,S-spiro-Pro-Ile)
<220>
   <221> MOD_RES
   <222> (3)
   <223> Trp is D-2-alkyl-Trp
<220>
   <221> MOD_RES
   <222> (4)
   <223> Lys is D-Lys
<220>
   <221> MOD_RES
   <222> (6)
   <223> Phe is D-Phe
<400> 36
   Pro Ile Trp Lys Trp Phe Lys
   1 5
<210> 37
   <211> 4
   <212> PRT
   <213> Artificial Sequence
<220>
   <223> Description of Artificial Sequence:synthetic peptides
<220>
   <221> MOD_RES
   <222> (1) .. (2)
   <223> Pro-leu is S,S-spiro-(Pro-Leu)
<220>
   <221> MOD_RES
   <222> (3)
   <223> Trp is D-2-alkyl-Trp
<220>
   <221> MOD_RES
   <222> (4)
   <223> Lys is D-HNH-(SO2CH3)-Lys
<400> 37
   Pro Leu Trp Lys
   1
<210> 38
   <211> 5
   <212> PRT
   <213> Artificial Sequence
<220>
   <223> Description of Artificial Sequence:synthetic peptides
<220>
   <221> MOD_RES
   <222> (1)
   <223> Trp is HAIC-D-2-alkyl-Trp
<220>
   <221> MOD_RES
   <222> (2)
   <223> Lys is D-Lys
<220>
   <221> MOD_RES
   <222> (4)
   <223> Phe is D-Phe
<400> 38
   Trp Lys Trp Phe Lys
   1 5
<210> 39
   <211> 5
   <212> PRT
   <213> Artificial Sequence
<220>
   <223> Description of Artificial Sequence:synthetic peptides
<220>
   <221> MOD_RES
   <222> (1)
   <223> trp is ATAB-D-2-alkyl-Trp
<220>
   <221> MOD_RES
   <222> (2)
   <223> Lys is D-Lys
<220>
   <221> MOD_RES
   <222> (4)
   <223> Phe is D-Phe
<400> 39
   Trp Lys Trp Phe Lys
   1               5